# EUROPEAN PATENT APPLICATION

(11) **EP 2 138 198 A1**
(43) Date of publication of application: **30.12.2009**
(21) Application number: 09251651.7
(22) Date of filing: 26.06.2009
(51) Int. Cl.: A61M 5/142, B81C 1/00

(54) **Fluidic capillary chip for regulating drug flow rates of infusion pumps**

(30) Priority: 27.06.2008 US 147605
(71) Applicant: Codman Neuro Sciences Sarl, 2400 Le Locle (CH)
(72) Inventor: Bork, Toralf, 2000 Neuchatel (CH); Bianchi, François, 1920 Martigny (CH)
(74) Representative: Small, Gary James

(57) **Abstract**

An erosion-resistant capillary chip for use with in an infusion pump that is made from a silicon substrate (102) having a first surface that includes a micro groove (118) etched therein and a glass plate (108) laminated to the first surface. The glass plate covers the micro groove so that a micro fluid conduit is created. The glass plate includes an inlet bore (114) that connects with the micro fluid conduit and the silicon substrate includes an outlet bore (124) that connects with the micro fluid conduit so that a drug solution entering the inlet bore from the infusion pump may pass through the micro fluid conduit at a restricted flow rate to the outlet bore and thereafter to a target site of a patient. The micro groove includes a passivation layer (126) made from silicon nitride or silicon carbide that protects the micro groove against erosion from passing fluids having high basic or high acidic pH levels. A method for making the capillary chip is disclosed, as well as an infusion pump incorporating the improved capillary chip.

## Description

### BACKGROUND OF THE INVENTION

### Field of the Invention

The present invention generally relates to implantable infusion pumps used to administer a controlled flow of drug into a patient, and more particularly, to improvements to micro capillary restrictor chips used to control the rate of flow in such devices.

### Description of the Related Art

Medication infusion devices reached commercial and medical acceptance decades ago for administering a controlled amount of a drug into a patient over an extending period of time. Earlier units were typically secured to the patient outside the body, but eventually, the components and technology that make up these devices evolved to a size and shape that permitted them to be implanted and operate directly within the patient's body during the course of treatment.

These implanted infusion pumps generally fall into two categories of operation, programmable devices and constant flow devices.

### Programmable Pumps

Currently commercially available programmable implantable infusion pumps typically employ a motor to drive a peristaltic pump. The rotational speed of the motor is controlled by programmable control circuitry so that the dosage rate can be varied to suit the needs of a particular patient. Other examples of programmable infusion pumps are shown in U.S. Pat. Nos. 4,077,405; 4,443,218; 4,447,224; and 4,714,462.

### Constant Flow Pumps

Constant flow infusion pumps are used in a variety of medical applications, for example, to dispense chemotherapy drugs at a relatively constant rate for long periods of time. These pumps rely on a liquid/vapor equilibrium to maintain constant pressure to the drug. In order to create a constant and controlled flow rate to the drug solution, the pressurized drug solution flows through a capillary tube restrictor prior to reaching the patient's body. Examples of such constant flow infusion pumps can be found in U.S. Pat. Nos. 3,731,681; 4,193,397; and 4,258,711.

The constant flow infusion pumps are implanted in the patient's body and are intended to remain there for a prolonged period. The implanted pumps include internal reservoirs that can be refilled with an infusate without the need for removal from the patient. Refilling is achieved by injecting additional infusate through a penetrable septum and into fluid communication with the onboard reservoir. As the infusate is injected into the storage chamber, the chamber expands and pressurizes a sealed quantity of gas. This effectively recharges the system and allows the sealed gas to again exert pressure on the newly filled reservoir within the device.

### Capillary Tube Restrictor

As mentioned above, the flow of the drug solution is dispensed from the reservoir of the infusion pump at a constant and controlled rate through the use of a capillary tube restrictor. In the past, the restrictor consisted of a single stainless steel or glass tube having a relatively large internal diameter (0.003-0.004 inches) and often extending a length exceeding 40 feet (typically wrapped around the implantable device). One such device is shown in U.S. Pat. No. 3,731,681 of Blackshear et al. and uses a stainless steel tube 50 feet long. Owing to the relatively large internal diameter, the long length of the tube is required to achieve the desired slow flow rate of the infusate.

Of course, such long lengths of capillary tubing also increased the space requirement and overall weight of the system. In the context of an implantable device, these two properties are significant drawbacks in that they limit areas where implantation may occur and provide a degree of user discomfort. Moreover, the actual measuring, calibrating and trimming of long tube lengths is labor intensive and requires a considerable amount of time.

### The Capillary Chip:

The problems associated with the construction of the above described capillary tube restrictor led to the development of the capillary chip, such as that shown in U.S. 4,537,680 of Barth. The capillary chip consists of an extremely small passage formed between two plates. A common method of making a capillary chip is to etch a serpentine or circuitous groove into the surface of a silicon substrate (a first plate). Since silicon is used here, the groove can be formed using conventional semiconductor processing techniques. Once the groove is formed, a glass or silicon plate (the second plate) is then bonded to the silicon to effectively cover the groove and thereby create a long capillary. Owing to the advanced etching techniques developed by the semiconductor industry, the resulting capillary can be formed with an extremely accurate and consistent cross-sectional area measured in square microns. The smaller the cross-sectional area of the capillary, the shorter the length required to achieve a given flow rate of a given liquid with a given viscosity and a given pressure drop, resulting in a compact flow-regulating structure. This is of course a desirable feature in designing compact and efficient infusion devices intended to be implanted into and carried by the body of a patient.

The capillary chip structure thus described is effective at providing short term reliable flow resistance to any of several commonly infused drugs and chemicals.

An object of the invention is to provide a capillary chip type infusion pump that provides protection against corrosion within micro fluid conduits of the capillary chip.

### Summary of the Invention

An erosion-resistant capillary chip, for use with in an infusion pump in accordance with the present invention that is made from a laminate of a silicon substrate and a glass plate. The silicon substrate includes a first surface that has a micro groove etched therein. The glass plate covers the micro groove so that a micro fluid conduit is created between the substrate and the glass plate. The glass plate includes an inlet bore that connects with the micro fluid conduit and the silicon substrate includes an outlet bore that connects with the micro fluid conduit so that a drug solution entering the inlet bore from the infusion pump may pass through the micro fluid conduit at a restricted flow rate to the outlet bore and thereafter to a target site of a patient. The micro groove includes a passivation layer made from silicon nitride or silicon carbide that protects the micro groove against erosion from passing fluids having high basic or high acidic pH levels. A method for making the capillary chip is also disclosed, as well as an infusion pump incorporating the improved capillary chip.

The accompanying drawings show examples of embodiments of the present invention. They illustrate how the invention achieves the above stated advantages and objectives.

### Brief Description of the Drawings

Fig. 1, labeled PRIOR ART is a sectional side view of an exemplary infusion pump showing details of a pressure bladder, a filter, a capillary chip and a bolus septum to help describe the present invention;
Fig. 2 is a perspective transparent view of a capillary chip according to the present invention showing details of a glass plate, a silicon substrate, an inlet, an outlet, a capillary and a protective layer; and
Fig. 3 is a sectional side illustrative view of a capillary chip assembly, according to the present invention, showing side view details of a glass plate, a silicon substrate, an inlet, an outlet, a capillary and a protective layer.

### Detailed Description of the Preferred Embodiments

By way of overview and introduction, the present invention concerns improvements in the capillary chip assembly used in certain types of infusion pumps which allow such infusion pumps to operate more reliably, and for a greater period of time over pumps using conventional capillary chips, while introducing a safe-guard to the patient.

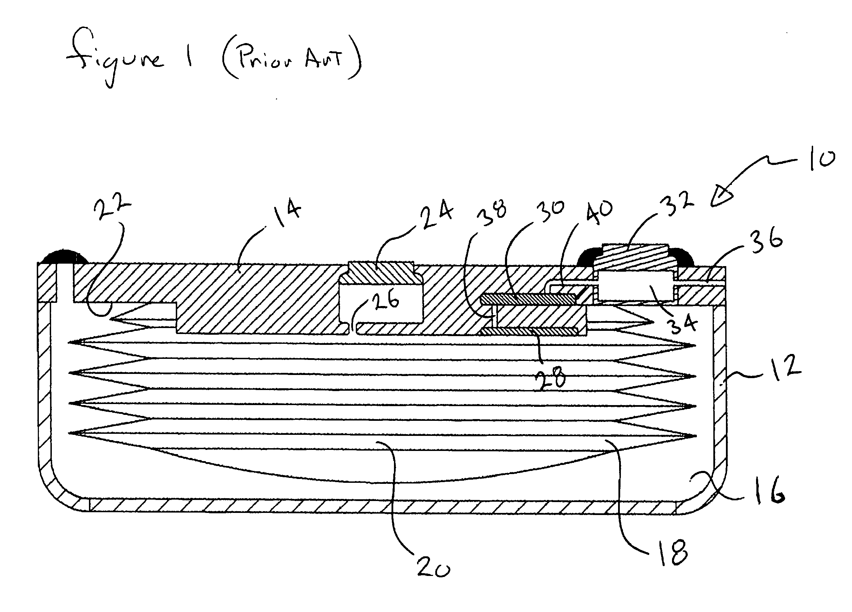

As a start, to help explain the present invention, a quick discussion of the structure and operation of a typical chip-capillary type infusion pump is in order. To this end, referring now to figure 1 (labeled PRIOR ART), a prior art infusion pump 10 is shown in section view, including a rigid housing 12 having a cover plate 14 and defining a gas-filled chamber 16. Located within the gas-filled chamber 16 is a flexible housing 18, which can expand and collapse as necessary within the rigid housing 12. This flexible housing 18 defines an infusate chamber 20 which is designed to hold the drug solution that is meant to be infused within the patient. Flexible housing 18, which is attached to a lower surface 22 of cover plate 14, is typically formed as an accordion-like bellows and is made from a strong bio-compatible material, such as titanium or stainless steel.

Cover plate 14 of this prior art infusion pump 10 includes a filling septum 24 which allows the filling of the drug solution through injection past the septum and then by way of an interposed filling conduit 26. Cover plate 14 further includes a filter 28, a capillary chip 30, a bolus septum 32, a bolus cavity 34 and finally, an outlet conduit 36. Filter 28 is positioned so that it is in fluid communication with both the drug solution of the infusate chamber 20 and also capillary chip 30 (via passage 38). Filter 28 is used to protect the capillary chip 30 from becoming clogged from any impurities that may be unintentionally suspended within the drug solution that is stored in the infusate chamber 20. As described above, the capillary chip 30 is used to regulate the flow of the passing drug solution to a controlled and predetermine flow rate (usually very slow rate of flow). The chip-capillary is selected to provide the patient with an accurate amount of drug solution over a period of time following a specific regimen determined by a physician. A passage 40 connects the outlet of capillary chip 30 and bolus cavity 34. Bolus septum 32 and bolus cavity 34 allow a physician to administer a predetermined amount of drug solution, as necessary, from a syringe directly to the outlet conduit 36 and eventually to a target site (not shown) within the patient, by-passing the flow-regulation of the capillary chip 30. Outlet conduit 36 connects with a catheter (not shown), which carries the drug solution from the infusion pump 10 to the target site located within the patient.

In operation of the PRIOR ART infusion pump 10 described above, an appropriate drug solution to be infused into the patient over a duration of time is injected into the infusate chamber 20 using a syringe to pierce filling septum 24. Prior to implanting the pump into the patient and prior to filling the infusate chamber 20, a propellant, such as n-butane is injected into the gas-filled chamber 16 of rigid housing 12 so that the propellant becomes sealed therein. As the drug solution is injected into the infusate chamber 20, the bellows that makes up the chamber expands to accommodate the increasing volume of the entering solution. As the filling continues and the bellows expands within sealed rigid housing 12, the relative pressure exerted by the propellant on the bellows increases steadily until the filling stops. The drug solution located within the infusate chamber 20 will now remain pressurized until all of the infusate has been dispensed into the patient.

The pressurized propellant and the drug solution within the infusate chamber 20 are at a greater pressure than the pressure measured at the target site within the patient so that the propellant pressure within the housing 12 effectively forces the drug solution through any path of least resistance to try to equilibrate the two pressures. In this case, the path begins by passes through filter 28, passage 38 and into the opening of capillary chip 30. Capillary chip 30 is essentially a micro passage through which the fluid must pass and, owing to the resulting friction created as the drug solution is forced through the micro passage (capillary), the drug solution slows its rate of flow tremendously before exiting the capillary chip and entering bolus cavity 34 by way of passage 40. The exact influence the capillary chip has on the flow rate of the drug solution depends on the shape and size of the micro passage and its length, as well as the viscosity of the drug solution and the fluid pressure differential between the entry opening of the capillary chip 30 and its exit.

Finally, the drug solution, now at a controlled rate of flow, passes through the bolus cavity 34 and the outlet passage 36 to the target site within the patient by way of the catheter (not shown).

The capillary chip of the prior art is a very delicate structure whose exacting dimensions must be maintained throughout the life of the infusion pump. Unfortunately, the working environment of these chip capillaries is less than predictable and the drug solutions that continuously pass through them (albeit very slowly) come into direct contact with the relatively sensitive silicon oxide material. This contact can lead to erosion of the delicate micro passageway located within the silicon structure. Once the passageway begins to erode, its cross-sectional area will increase and its exacting control of the flow rate of the passing drug solution will similarly erode, resulting in a faster flow rate. This unpredictable increase in drug infusion can easily result in a drug overdose condition, putting the patient's health at risk.

This corrosive action by the drug solution is a result of the pH value of the drug solution changing from a safe range, usually between 4 and 8 and becoming either strongly acidic or strongly basic. The pH value of drug solutions can vary over time as the stored solution remains within the infusate chamber 20, in some cases for as long as 150 days. As mentioned above, although the initial pH value of a drug solution measured prior to filling the pump may be within an accepted, safe range, the pH value may drift over time from this range as the drug remains in the pump waiting to be infused. For example, the drug morphine is known to degrade overtime resulting in a decrease of its measured pH value, while the drug baclofen has been shown to increase in pH value over time. Also, the drug solution can be prepared improperly during a refill, for example, which can change the pH of the entire batch located within the infusate chamber 20. Regardless of how the drug solution becomes corrosive, the end result is that the integrity of the prior art capillary chip cannot be ensured and the health of the patient may even become threatened.

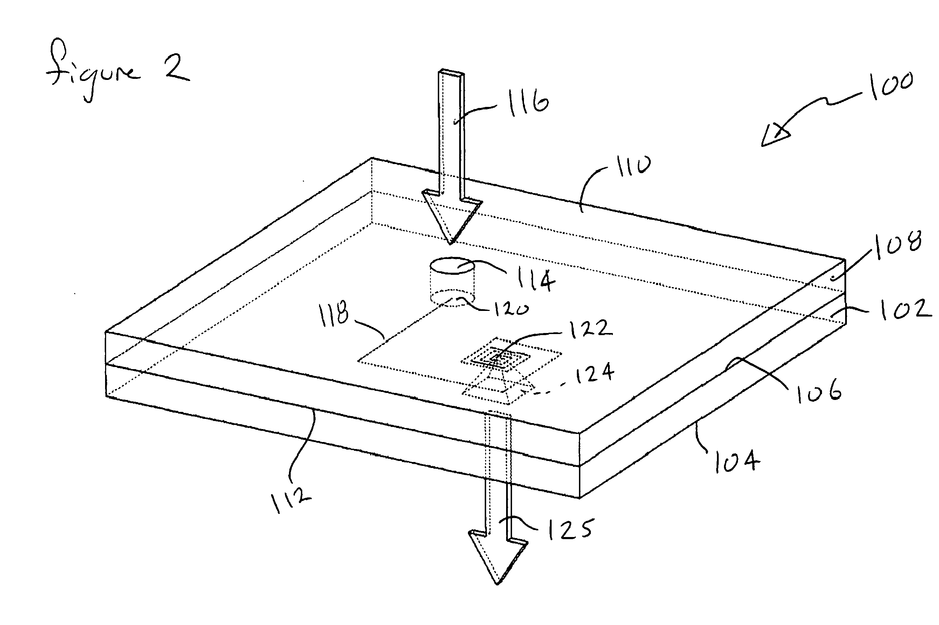

According to the present invention, referring to Fig. 2, a capillary chip100 is shown including a substrate 102 having an outer surface 104 and an inner surface 106 and a cover plate 108, having an outer surface 110 and an inner surface 112. The components of this figure are shown transparent to help explain the details of the invention. As shown in the figure, cover plate 108 includes a bore 114 which extends from outer surface 110 and inner surface 112 and which functions as the inlet to the capillary chip, as illustrated by arrow 116. Substrate 102 includes a micro-groove 118 that has been etched into the inner surface 106. The shape of the micro-groove (planar view) is preferably a spiral, starting at a start point 120 and ending at an end point 122. A bore 124 is formed through substrate 102 at end point 122 and is generally funnel-shaped, opening up at inner surface 106. This bore 124 functions as an outlet to the drug solution, as illustrated by arrow 125.

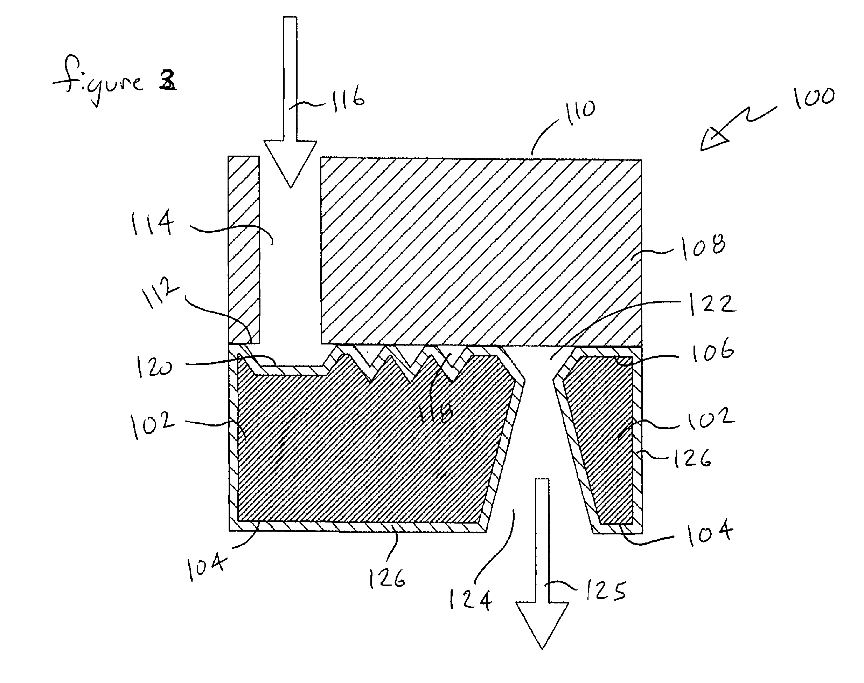

Referring to Fig. 3, substrate 102 is preferably made from monocrystalline silicon and includes a protection layer 126 that covers and protects the entire substrate, but most importantly covers and protects the micro groove 118 from erosion by preventing the passing drug solution from direct contact with the more susceptible silicon substrate 102. Protective layer 126 is preferably a passivation layer in that it is made from a corrosion-resistant material that is strongly bonded to the silicon substrate, being resistant to a higher pH range, both acidic and basic.

To manufacture the above described capillary chip 100, conventional semiconductor fabrication techniques can be employed. As understood by those skilled in the art, micro groove 118, for example, can be formed by first growing a layer of silicon oxide on the surface of the monocrystalline silicon substrate 102.

As is understood by those skilled in the art, silicon oxide is commonly used in the construction of semiconductor devices. Briefly, there are three commonly adopted methods of forming a silicon oxide film on the surface of a silicon substrate (1) chemical vapor deposition; (2) high temperature oxidation; and (3) spin-on glass.

One of these methods may be used to create the required oxidation layers of the present invention. The preferred method would be known to one of ordinary skill in the art of semiconductor manufacturing and the particulars of this method are beyond the scope of this patent application.

Using a standard photolithography and etching process, a mask pattern for the micro groove 118 (the capillary) is transferred to the silicon dioxide layer with the oxide in the mask pattern being removed. Thereafter, using the remaining oxide on the surface as a mask, the micro groove 118 is etched in the surface. Preferential etchants such as a solution of potassium hydroxide can be used in the etching process to create the micro groove 118 at a predetermined depth. As is well known to those skilled in semiconductor fabrication processes, isotropic etchants such as mixtures of hydrofluoric acid and nitric acid or dry reactive ion etching techniques may also be used to etch away at the silicon substrate where the silicon oxide mask allows.

After micro groove 118 is formed, the remaining oxide is removed from the silicon surface by a suitable etchant which does not otherwise damage the silicon substrate 102. Similar etching techniques can be used to form funnel-shaped bore 124 in silicon substrate 102 at the desired location (at the end point 122 of micro groove 118).

As a final step, according the present invention, protection layer 126 is applied by CVD (chemical vapor deposition) or PVD (physical vapor deposition) to the silicon oxide layer and any exposed silicon substrate. Protection layer 126 is preferably made from silicon carbide or silicon nitride because it has been determined in experimentation that both of these materials are substantially more resistant to corrosion than silicon oxide in similar conditions. The protective layer 126 covers the entire silicon substrate including the newly formed micro groove 118 and bore 124.

The engineered etched depth of the micro groove 118 and the thickness of the final protective layer 126 have been calculated so that the resulting micro groove 118 with protective layer 126 has the desired dimensions and cross-sectional area. Owing to the etching and coating process described above, the micro groove 118 will likely have a triangular cross-sectional shape and will be about 41.5 microns across at the widest point and about 30 microns in depth, of course depending on the desired flow rate and the particulars of the drug solution. The thickness of the protective layer will be in the range of 100nm to 3 microns, depending on the desired flow rate, the size and shape of the micro groove 118 and other particulars of the capillary chip structure and use.

Cover plate 108 is preferably made from an appropriate precision ground and polished glass. Prior to securing cover plate 108 to substrate 102, bore 114 can be formed into cover plate at the desired and predetermined location using any appropriate techniques, such as ultrasonic drilling. Once the bore is formed, inner surface 112 of cover plate 108 is bonded to the protective layer 126 of substrate 102 (inner surface 104) so that bore 114 of cover plate 108 aligns with start point 120 of micro groove 118 of silicon substrate 102. Cover plate 14 may be bonded to the protective layer 126 of substrate 102 using a conventional anodic bonding process, thereby covering the etched and protected micro groove 118. When cover plate covers micro-groove 118, the capillary of the capillary chip 100 is formed.

Providing the above described protective layer 126 to the silicon substrate using any appropriate technique, preferably CVD (chemical vapor deposition) or PVD (physical vapor deposition), as known to those skilled in the art should pose no difficulties in batch production, thereby reducing the cost of the structure, and known techniques for fabricating the structure in silicon permit accurate reproduction.

Since bore 114 aligns with start point 120 of micro groove 118 and end point 122 of micro groove aligns with bore 124, in operation drug solution may enter bore 114, pass through the entire length of micro groove 118 and exit through bore 124 without directly contacting any silicon portion of the silicon substrate 102, contacting only the more resistant protective layer 126. The resulting capillary chip 100 will allow drug solutions having more extreme pH values to pass through micro groove 118 without eroding silicon substrate 102 and without enlarging micro groove 118. This will effectively discourage, if not prevent accidental overdose to the patient. Since the protected capillary chip 100 will be able to handle drug solutions having a higher range of pH values, infusion pumps using this type of capillary chip will now be able to safely pump a wider variety of drugs, perhaps opening the door to new stronger drug therapies that would have otherwise been avoided by the manufacturers of prior art infusion pumps using non-protected chip capillaries, such as capillary chip 30, of Figure 1 and described above.

Applicants contemplate using any material or combination of materials to create the protective layer 126, as long as the protective layer provides greater corrosion resistant over silicon oxide.

While the invention has been described with reference to a specific embodiment, the description is illustrative of the invention and is not to be construed as limiting the invention. For example, cover plate 108 may be made from a semiconductor or metal body instead of an insulator as described above and silicon substrate 102 may be made from glass (wherein a protective layer 126 may not be required and wherein hydrofluoric acid may be used as the etchant to form the micro-groove 118). Also, bores 114, 124 which provide access to the start and end points of micro groove 118 can be formed entirely from either the cover plate 108, the semiconductor substrate 102 or from the edge of the substrate.

Other modifications and applications may occur to those skilled in the art without departing from the true spirit and scope of the invention as defined by the appended claims.

## Claims

1. A capillary for use in an infusion pump, comprising:
a substrate having a first surface;
a channel on said first surface extending between a start point and an end point;
a passivation layer applied to said first surface within said channel;
an outlet bore in said substrate positioned at said end point;
a cover affixed to said first surface so that said channel is covered, thereby defining a fluid conduit, said cover including an inlet bore located in alignment with said start point of said channel so that said fluid conduit is in fluid communication with both said inlet bore and said outlet bore.

2. The capillary of claim 1, wherein said substrate is made from silicon or glass.

3. The capillary of claim 1, wherein said channel is V-shaped, rectangular-shaped, or semi-circular-shaped in cross-section.

4. The capillary of claim 2, wherein said substrate is made from silicon and wherein said channel is V-shaped or rectangular-shaped in cross-section.

5. The capillary of claim 4, wherein the passivation layer is made of a material having a greater corrosion resistance of silicon oxide.

6. The capillary of claim 2, wherein said substrate is made from glass and wherein said channel is semi-circular or rectangular in cross-section.

7. The capillary of claim 1, wherein said channel is rectangular in cross-section.

8. The capillary of claim 1 or claim 2 when said substrate is made from silicon, wherein said channel follows a serpentine path on said first surface between said start point and said end point.

9. The capillary of claim 1, wherein said channel follows a spiral path on said first surface between said start point and said end point.

10. The capillary of claim 8 when dependent upon claim 1, wherein said serpentine-shaped channel further follows a spiral path on said first surface between said start point and said end point.

11. The capillary of claim 1 or claim 2 when said substrate is made from silicon, wherein said passivation layer is made from silicon nitride.

12. The capillary of claim 1 or claim 2 when said substrate is made from silicon, wherein said passivation layer is made from silicon carbide.

13. The capillary of claim 1, claim 2 when said substrate is made from silicon, or claim 5 when said capillary is rectangular-shaped in cross-section, wherein said cover is made from glass.

14. The capillary of claim 1, claim 6, claim 7 or claim 8 when dependent upon claim 1, wherein said passivation layer covers the entire surface of said first face.

15. A capillary for use in an infusion pump, comprising:
a flat substrate of material having a first face and an opposing parallel second face;
a channel on said first face extending between a start point and an end point;
a passivation layer formed onto to said first face within said channel;
an outlet bore in said substrate positioned at said end point, said outlet bore extending between said first and second faces;
a cover having an inner face and a parallel outer face, said inner face of said cover being affixed to said first face of said substrate so that said channel is covered, thereby defining a fluid conduit which extends between said start point and said end point, said cover including an inlet bore located in alignment with said start point, said inlet bore extending between said inner and outer faces; and
so that said fluid conduit is in fluid communication with both said inlet bore and said outlet bore.

16. An infusion pump for infusing a drug solution to a target site of a patient, said pump being of the type that includes a pressurized supply of drug solution, a capillary chip comprising:
a substrate of material having a first face and an opposing second face;
a channel on said first face extending between a start point and an end point;
a passivation layer bonded to said first face within said channel;
an outlet bore in said substrate positioned at said end point, said outlet bore extending between said first and second faces;
a cover having an inner face and a parallel outer face, said inner face of
said cover being affixed to said first face of said substrate so that said channel is covered, thereby defining a fluid conduit which extends between said start point and said end point, said cover including an inlet bore located in alignment with said start point, said inlet bore extending between said inner and outer faces; and
wherein said inlet bore is positioned within said pump to be in fluid communication with said pressurized supply of drug solution and wherein said outlet bore is positioned to be in fluid communication with said target site of said patient, so that said drug solution may be forced through said inlet bore, said fluid conduit and out said outlet bore of said capillary chip to said target site of said patient.

17. The infusion pump of claim 16, wherein said substrate is made from silicon.

18. The infusion pump of claim 16, wherein said channel follows a serpentine/spiral or spiral-serpentine path on said first surface between said start point and said end point.

19. The infusion pump of claim 16 or claim 17, wherein said passivation layer is made from silicon nitride.

20. The infusion pump of claim 16 or claim 17, wherein said passivation layer is made from silicon carbide.

21. The infusion pump of claim 19 or claim 20, wherein said passivation layer covers the entire surface of said first face.

22. A method for making a capillary chip of the type used to restrict the flow rate of a drug solution of an infusion pump prior to the drug solution reaching a target site within a patient, said capillary chip including a silicon substrate having a first surface and an outlet bore and a cover plate having an inlet bore, the method comprising the steps of:
forming a groove within said first surface of said silicon substrate, said groove including walls and extending between a start point and said outlet bore;
applying a passivation layer to said first surface so that said passivation layer covers said walls of said groove; and
affixing said cover plate to said first surface of said substrate so that said inlet bore aligns with said start point and so that said groove defines a fluid conduit through which said drug solution may selectively pass without contacting said silicon substrate and whose rate may therein become reduced.

23. The method for making a capillary chip of claim 22, wherein said passivation layer is made from silicon carbide or silicon nitride.

24. The infusion pump of claim 16, wherein said infusion pump is a peristaltic type pump.
